# FASCICULE DE BREVET EUROPEEN

(11) **EP 0 582 506 B1**
(45) Date de publication et mention de la délivrance du brevet: **28.02.1996**
(21) Numéro de dépôt: 93401944.9
(22) Date de dépôt: 27.07.1993
(51) Int. Cl.: C07C 233/78, C07C 255/60, C07C 233/88, C07C 233/66, C07C 233/76, A61K 31/165, C07D 213/36, C07D 319/18, A61K 31/275, A61K 31/335, A61K 31/44

(54) **Dérivés de tétrahydronaphtalène leur préparation et leur application en thérapeutique**
Tetrahydronaphtalenderivate, ihre Herstellung und ihre therapeutische Verwendung
Tetrahydronaphtalene derivatives, their preparation and their application in therapy

(30) Priorité: 05.08.1992 FR 9209713
(43) Date de publication de la demande: 09.02.1994
(73) Titulaire: SYNTHELABO, F-92350 Le Plessis Robinson (FR)
(72) Inventeur: Williams, Paul, Howard, F-75011 Paris (FR); Hoornaert, Christian, F-75013 Paris (FR); MULLER Jean-Claude, 91390 Morsang S/Orge (FR)
(74) Mandataire: Thouret-Lemaitre, Elisabeth

(56) Documents cités:
- EP-A- 0 233 762
- EP-A- 0 352 613
- DE-B- 1 247 315

## Description

La présente invention a pour objet des dérivés de tétrahydronaphtalène, leur préparation et leur application en thérapeutique.

Les composés de l'invention répondent à la formule (I) 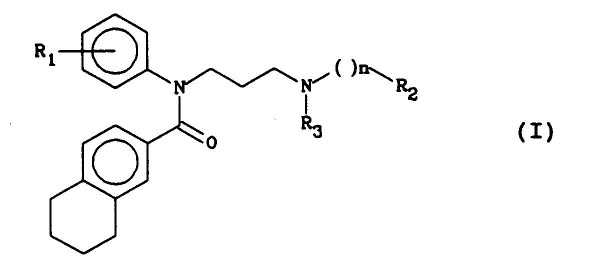
dans laquelle
R₁ représente soit un atome d'halogène, soit un groupe cyano, soit un groupe nitro, soit un groupe (C₁-C₆)alcoxy droit ou ramifié, soit un groupe (C₁-C₆)cycloalkyl(C₁-C₂)alcoxy, soit un groupe aryl(C₁-C₂)alcoxy,
R₂ représente soit un groupe (C₁-C₄)alkyle, soit un groupe aryle éventuellement substitué par un ou plusieurs groupes méthoxy, soit un groupe pyridinyle, soit un groupe benzodioxanyle,
R₃ représente soit un atome d'hydrogène, soit un groupe (C₁-C₂)alkyle, et
n = 0 à 3.

Les composés préférés de l'invention sont ceux répondant à la formule (I) dans laquelle
R₁ représente un groupe (C₁-C₆)alcoxy droit ou ramifié,
R₂ représente un groupe aryle substitué par un ou plusieurs groupes méthoxy,
R₃ représente un groupe (C₁-C₂)alkyle et
n = 2.

Parmi ces composés préférés, le composé de choix est le composé répondant à la formule (I) dans laquelle
R₁ représente un groupe 2-méthylpropoxy,
R₂ représente un groupe 3,4-diméthoxyphényle,
R₃ représente un groupe méthyle et
n = 2.

Les composés de l'invention peuvent se présenter sous forme de bases libres ou de sels d'addition à des acides pharmaceutiquement acceptables. Ces différentes formes font partie de l'invention.

On peut préparer les composés de l'invention pour lesquels R₃ représente un atome d'hydrogène selon le schéma 1 ci-dessous: 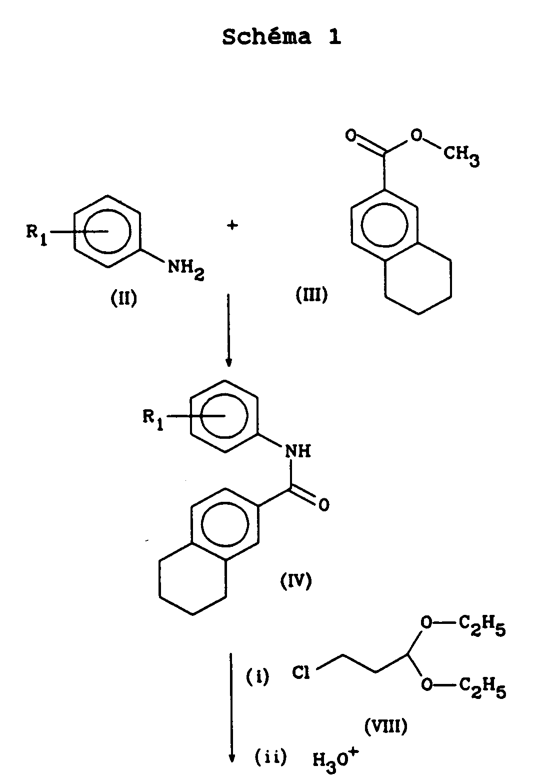 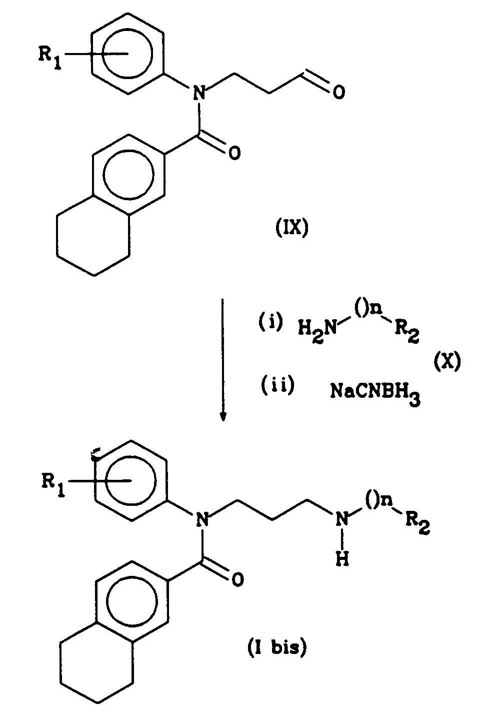
On fait réagir le 5,6,7,8-tétrahydronaphtalène-2-carboxylate de méthyle (III), avec une amine de formule (II) dans laquelle R₁ est tel que défini ci-dessus, pour obtenir un carboxamide de formule (IV) que l'on condense avec le 3-chloro-1,1-diéthoxypropane de formule (VIII), pour obtenir un carboxamide que l'on traite en milieu acide pour donner un carboxamide de formule (IX), composé que l'on condense avec une amine de formule (X) dans laquelle R₂ et n sont tels que définis ci-dessus, en présence de cyanoborohydrure de sodium, dans un solvant tel que le méthanol, pour obtenir un carboxamide de formule (I bis).

Conformément à l'invention, on peut préparer les composés de formule générale (I), dans laquelle R₃ représente un groupe (C₁-C₂)alkyle, selon le schéma 2 de la page suivante: 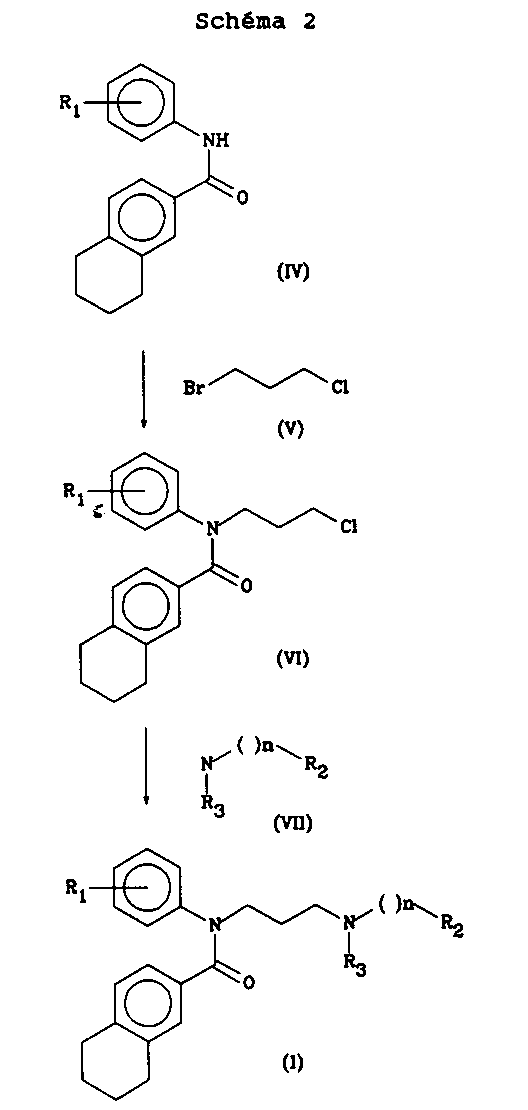
On condense le composé de formule (IV) avec du 1-bromo-3-chloropropane de formule (V), en présence d'une base telle que l'hydrure de sodium, pour obtenir un composé de formule (VI) que l'on fait réagir avec une amine de formule (VII) dans laquelle R₂, R₃ et n sont tels que définis ci-dessus, pour obtenir le composé de formule (I).

On prépare le 5,6,7,8-tétrahydronaphtalène-2-carboxylate de méthyle (III) à partir du 5,6,7,8-tétrahydro-2-naphtol, sur lequel on fait réagir de l'anhydride trifluorométhanesulfonique pour obtenir le trifluorométhanesulfonate de 5,6,7,8-tétrahydronaphtalèn-2-yle que l'on transforme en carboxylate de formule (III).
On peut préparer la benzènamine de formule (II) par différentes méthodes connues de l'homme du métier. Les autres produits de départ sont disponibles dans le commerce.

Les composés intermédiaires sont nouveaux et font également partie de l'invention. Ils répondent à la formule (XI) 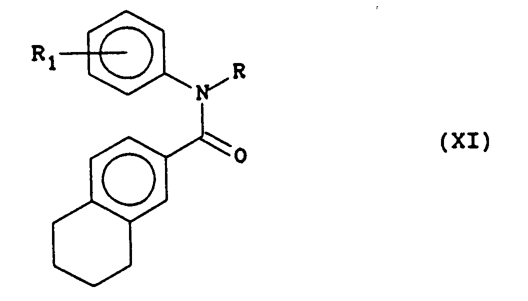
dans laquelle
R représente soit un atome d'hydrogène, soit un groupe 3-chloropropyle, soit un groupe 3-oxopropyle,
R₁ représente soit un atome d'halogène, soit un groupe cyano, soit un groupe nitro, soit un groupe (C₁-C₆) alcoxy droit ou ramifié, soit un groupe (C₁-C₆)cycloalkyl(C₁-C₂)alcoxy, soit un groupe aryl(C₁-C₂)alcoxy.

Les exemples qui suivent illustrent en détail la préparation de quelques composés selon l'invention. Les microanalyses élémentaires et les spectres IR et RMN confirment les structures des produits obtenus.

### Exemple 1

### N-[3-[[2-(3,4-diméthoxyphényl)éthyl]méthylamino]propyl]-N-[2-(2-méthylpropoxy)phényl]-5,6,7,8-tétrahydronaphtalène-2-carboxamide, oxalate

### 1.1. 1-(2-méthylpropoxy)-4-nitrobenzène

A 50 g (0,36 mole) de 4-nitrophénol, on ajoute 98,66 g (0,72 mole) de 1-bromo-2-méthylpropane, 74,63 g (0,54 mole) de carbonate de potassium et 250 ml de diméthylformamide. On chauffe le mélange à 100 °C, pendant 4 heures puis on évapore à sec. On reprend le résidu par 500 ml d'une solution de soude 1 N et on ajoute 200 ml d'éther. On récupère la phase organique et on la lave successivement trois fois par 100 ml de soude 1 N, puis trois fois par 200 ml d'eau et finalement par 100 ml d'une solution saturée de chlorure de sodium. On la sèche sur du sulfate de magnésium et on évapore à sec. On obtient 70 g de produit.

### 1.2. 4-(2-méthylpropoxy)benzènamine

Dans un appareil de Parr, on ajoute 22 g (0,11 mole) du composé obtenu en 1.1, 200 ml d'éthanol et de l'oxyde de platine. On réalise une hydrogènation catalytique pendant 3 heures à la température ambiante, sous une pression de 0,28 MPa. On filtre le mélange sur célite et on lave la célite par de l'éthanol. On évapore le filtrat à sec et on obtient 18 g de produit que l'on purifie par distillation. On obtient 33,5 g de produit pur.
Point d'ébullition = 86-92 °C à 200 Pa

### 1.3. trifluorométhanesulfonate de 5,6,7,8-tétrahydronaphtalèn-2-yle

Dans un ballon de 1 litre, on met 17,5 g (0,12 mole) de 5,6,7,8-tétrahydro-2-naphtol, 28,86 g (0,24 mole) de 4-diméthylaminopyridine, 20,64 ml (0,18 mole) de 2,6-diméthylpyridine et 400 ml de dichlorométhane. On refroidit le mélange, à - 30°C, sous argon. On ajoute goutte à goutte, 50 g (0,18 mole) d'anhydride trifluorométhanesulfonique dans 120 ml de dichlorométhane, puis on laisse revenir à la température ambiante. On ajoute ensuite 500 ml de dichlorométhane et on lave successivement par 200 ml d'acide chlorhydrique 1 N, trois fois 200 ml d'eau, 200 ml d'une solution saturée en hydrogénocarbonate de sodium, deux fois 200 ml d'eau et 200 ml d'une solution saturée en chlorure de sodium. On sèche sur du sulfate de sodium et on évapore à sec. On purifie le résidu par chromatographie sur colonne de gel de silice en éluant par de l'hexane.

### 1.4. 5,6,7,8-tétrahydronaphtalène-2-carboxylate de méthyle

Dans un ballon tricol de 1 litre, on solubilise 32,3 g (0,12 mole) du composé obtenu en 1.3, dans 345 ml de diméthylsulfoxyde et 230 ml de méthanol. On ajoute 35,21 ml (0,35 mole) de triéthylamine, 0,8 g (0,004 mole) d'acétate de palladium et 1,44 g (0,004 mole) de 1,3-bis(diphénylphosphino)propane. On fait barboter un courant de monoxyde de carbone pendant 5 minutes, puis on chauffe à 70°C, sous atmosphère de monoxyde de carbone, pendant 4 heures. Ensuite on ajoute 400 ml d'eau et on extrait deux fois avec 200 ml d'éther. On lave les phases éthérées avec 200 ml d'eau, on les sèche sur du sulfate de magnésium et on évapore à sec. On purifie le résidu par chromatographie sur colonne de gel de silice en éluant avec un mélange dichlorométhane/hexane (4/6).

### 1.5. N-[2-(2-méthylpropoxy)phényl]-5,6,7,8-tétrahydronaphtalène-2-carboxamide

Dans 10 ml de diméthylsulfoxyde contenant une goutte de méthanol, sous argon et sous agitation magnétique, on met 1,3 g (0,03 mole) d'une suspension d'hydrure de sodium à 50 %. On laisse sous agitation pendant 10 minutes et on ajoute 2,6 g (0,016 mole) de 4-(2-méthylpropoxy)benzèneamine obtenue en 1.2. On laisse sous agitation pendant quelques minutes, puis on ajoute goutte à goutte 2,5 g (0,013 mole) du composé obtenu en 1.4, en solution dans 10 ml de diméthylsulfoxyde. On laisse sous agitation pendant 4 heures à la température ambiante. Ensuite, on ajoute successivement, 200 ml d'eau, 100 ml d'éther et 100 ml d'acétate d'éthyle. On sépare la phase organique, on la lave successivement par 100 ml d'eau, 100 ml d'acide chlorhydrique 1 N, deux fois 50 ml d'eau et 100 ml de chlorure de sodium. On la sèche sur du sulfate de magnésium et on évapore à sec.

### 1.6. N-(3-chloropropyl)-N-[2-(2-méthylpropoxy)phényl]-5,6,7,8-tétrahydronaphtalène-2-carboxamide

Dans un ballon, sous agitation et sous azote, on ajoute 2,58 g (0,008 mole) du composé obtenu précédemment en 1.5, en solution dans 13 ml de diméthylformamide. On ajoute lentement, 0,5 g (0,01 mole) d'une suspension d'hydrure de sodium à 50 %, on refroidit à 0°C et on ajoute goutte à goutte 1,88 g (0,01 mole) de 1-bromo-3-chloropropane. On laisse remonter à la température ambiante et on laisse sous agitation pendant 4 heures. On refroidit la solution, on ajoute 100 ml d'eau et 100 ml d'éther. On agite, on récupère la phase organique et on extrait la phase aqueuse avec 100 ml d'éther. On rassemble les phases organiques qu'on lave successivement avec deux fois 50 ml d'eau, 50 ml d'acide chlorhydrique 1 N, deux fois 50 ml d'eau et 50 ml d'une solution saturée en chlorure de sodium. On sèche sur du sulfate de magnésium et on évapore à sec.
On obtient 3,3 g de produit.

### 1.7. N-[3-[[2-(3,4-diméthoxyphényl)éthyl]méthylamino] propyl]-N-[2-(2-méthylpropoxy)phényl]-5,6,7,8-tétrahydronaphtalène-2-carboxamide, oxalate

On chauffe à 80°C, pendant 5 heures, un mélange de 2 g (0,006 mole) du composé obtenu précédemment en 1.6, 1,54 g (0,01 mole) de carbonate de potassium, 0,93 g (0,006 mole) d' iodure de potassium et 1,1 g (0,006 mole) de 3,4-diméthoxy-*N*-méthylbenzèneéthanamine. On refroidit la solution et on ajoute 100 ml d'eau et 100 ml d'éther. On récupère la phase organique et on réextrait la phase aqueuse par 100 ml d'éther. On réunit les phases organiques et on les lave par trois fois 100 ml d'eau. On les sèche sur du sulfate de magnésium et on évapore à sec. On purifie le résidu par chromatographie sur colonne de gel de silice en éluant par un mélange dichlorométhane/méthanol (9/1). On obtient 0,7 g de produit.

On prépare l'oxalate en ajoutant un équivalent d'acide oxalique.
Point de fusion = 114-116 °C

### Exemple 2

### N-[2-(2-méthylpropoxy)phényl]-N-[3-(2-phényléthyl)amino]-propyl]-5,6,7,8-tétrahydronaphtalène-2-carboxamide, fumarate

### 2.1. N-(3,3-diéthoxypropyl)-N-[2-(2-méthylpropoxy)phényl]-5,6,7,8-tétrahydronaphtalène-2-carboxamide

A 1,6 g (0,033 mole) d'une suspension d'hydrure de sodium à 50 % dans 20 ml de diméthylsulfoxyde, on ajoute, sous azote, 9,7 g (0,03 mole) du composé obtenu en 1.5, en solution dans 25 ml de diméthylformamide. On chauffe pendant 1 heure 30 à 50°C et on ajoute 5,5 g (0,03 mole) de 3-chloro-1,1-diéthoxypropane. On chauffe à 100°C, dans un bain d'huile, pendant 8 heures. Puis on verse sur un mélange glace + eau et on extrait par de l'éther. On lave la phase organique avec de l'eau puis avec une solution saturée en chlorure de sodium. On sèche sur du sulfate de magnésium et on purifie l'huile résiduelle, par chromatographie sur colonne de gel de silice, en éluant par un mélange hexane/acétate d'éthyle (4/1).
On obtient 4,3 g de produit.

### 2.2. N-[2-(2-méthylpropoxy)phényl]-N-(3-oxopropyl)-5,6,7,8-tétrahydronaphtalène-2-carboxamide

On chauffe, au bain d'huile, à 50°C, pendant 4 heures, 4,3 g (0,009 mole) du composé obtenu précédemment en 2.1, dans 100 ml d'acide chlorhydrique. Ensuite, on neutralise le milieu réactionnel par de l'hydrogénocarbonate de sodium et on extrait par de l'éther. On récupère la phase organique et on la lave par une solution saturée en chlorure de sodium. On la sèche sur du sulfate de magnésium et on évapore à sec. On purifie le résidu par chromatographie sur colonne de gel de silice, en éluant par un mélange d'acétate d'éthyle/hexane (2/3).
On obtient 2,7 g de produit.

### 2.3. N-[2-(2-méthylpropoxy)phényl)-N-[3-[(2-phényléthyl) amino]propyl]-5,6,7,8-tétrahydronaphtalène-2-carboxamide, fumarate

A une solution de 0,947 g (0,003 mole) du composé obtenu précédemment en 2.2, dans 25 ml de méthanol, sous azote, on ajoute, 0,25 g de tamis 3 Å puis 1,5 g (0,013 mole) de benzèneéthanamine puis 0,625 ml d'acide chlorhydrique 6 N et 0,12 g (0,002 mole) de cyanoborohydrure de sodium. On laisse le mélange, à la température ambiante, pendant une nuit. Ensuite, on ajoute 1 ml d'éthanol chlorhydrique et 0,4 g de cyanoborohydrure de sodium et on laisse sous agitation pendant 3 heures. On rajoute de l'éthanol chlorhydrique, on évapore à sec et on reprend le résidu par de l'eau. On neutralise le milieu avec du carbonate de sodium et on extrait par un mélange éther/acétate d'éthyle. On récupère la phase organique, on la lave à l'eau, on la sèche sur du sulfate de magnésium et on évapore à sec.
On obtient 0,85 g de produit.
On prépare le fumarate en ajoutant un équivalent d'acide fumarique et en recristallisant dans de l'alcool isopropylique.
Point de fusion = 148-150 °C

Le tableau suivant illustre les structures chimiques et les propriétés physiques de quelques composés selon l'invention.

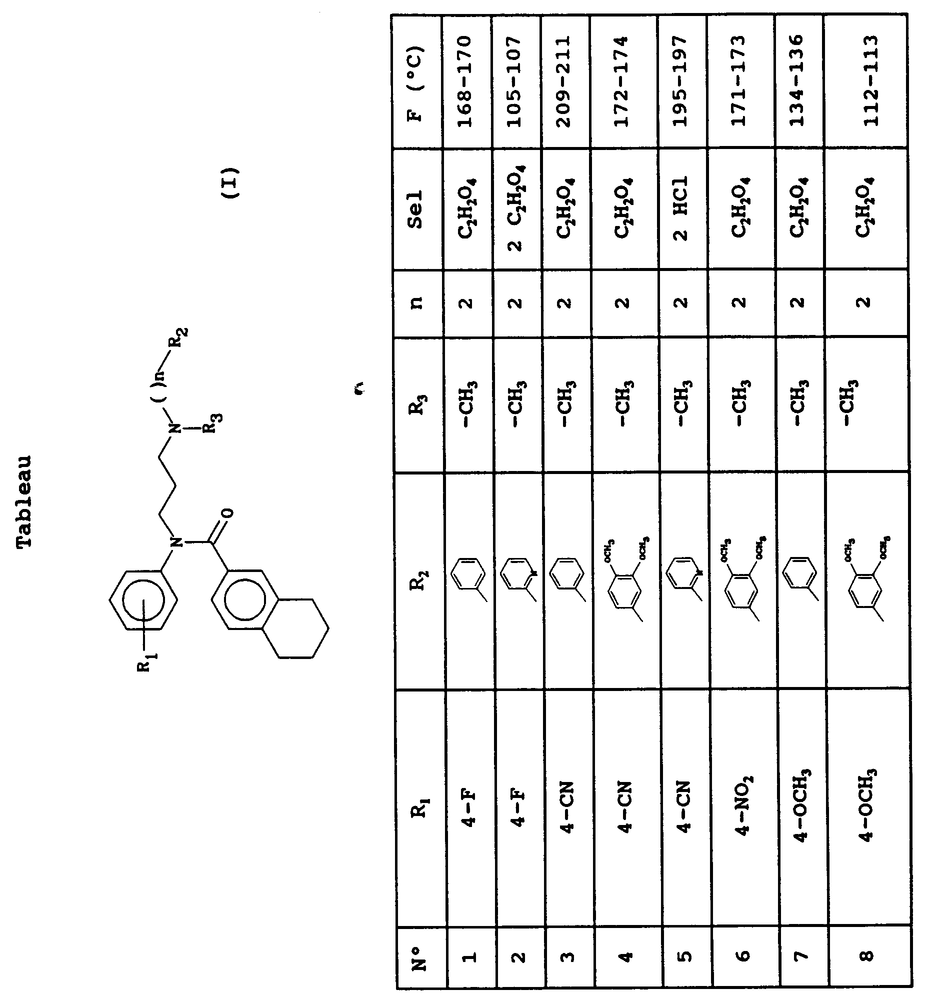

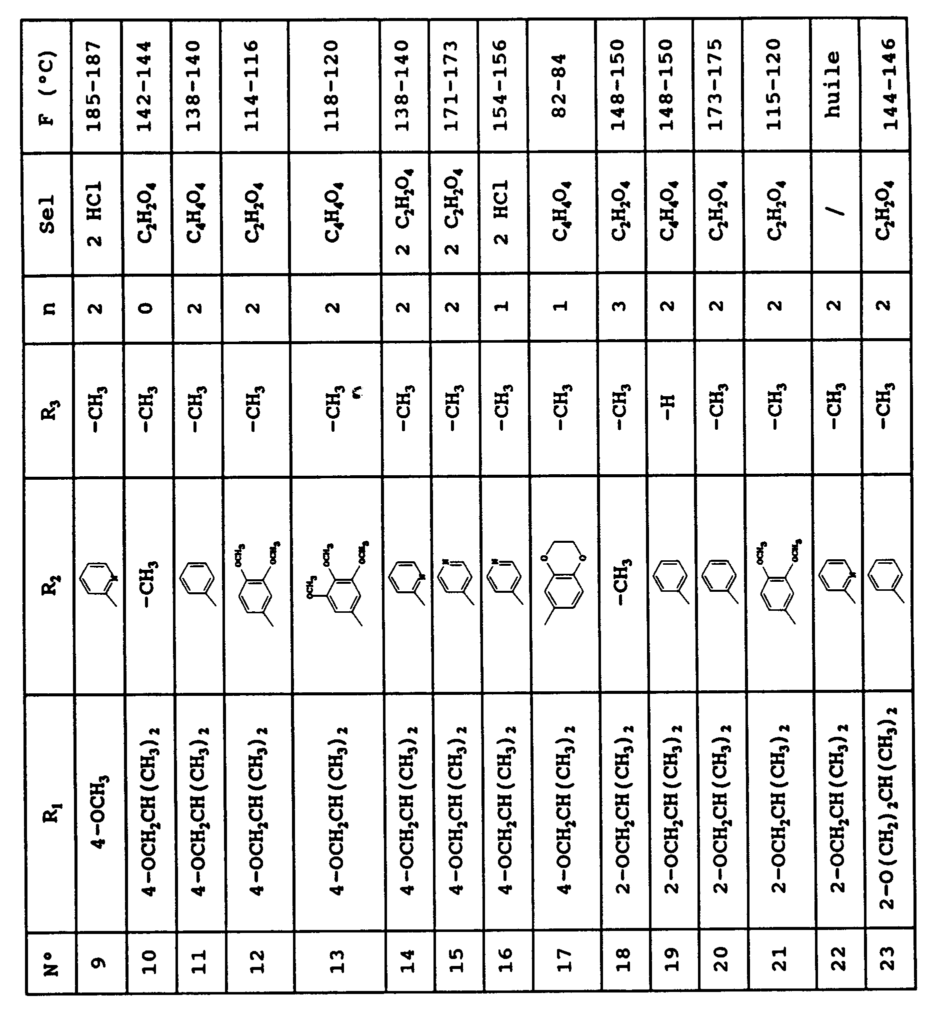

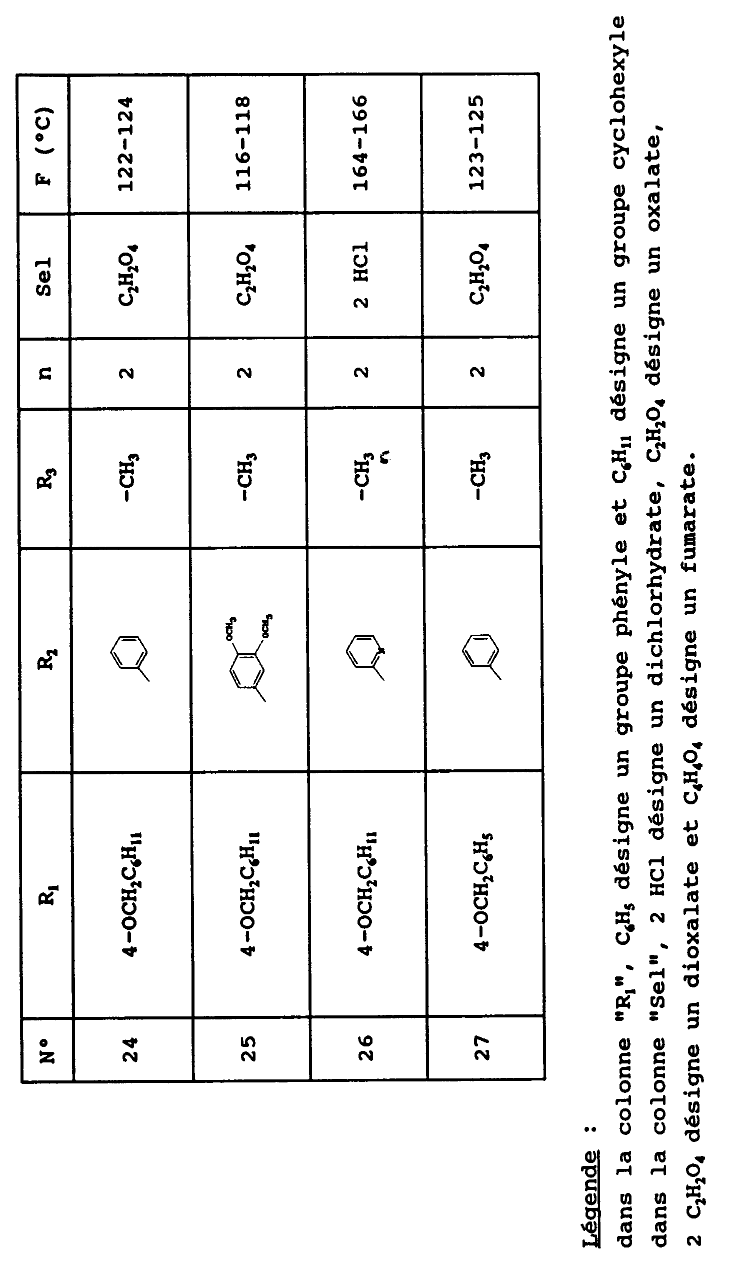

Les composés de l'invention ont fait l'objet d'essais pharmacologiques qui ont montré leur intérêt comme substances actives en thérapeutique.

### Inhibition de l'entrée du calcium induite par KCl dans des coupes de cortex de rat immature

On utilise des rats Sprague-Dawley, mâle ou femelle, âgés de 8 jours. Après dislocation cervicale, on excise le cerveau et on prépare des coupes de cortex pariètal.
La concentration intracellulaire en calcium ([Ca²⁺]ᵢ) est mesurée selon la technique décrite dans J. Pharm. Exp. Ther., **261**, (1992), page 324-330. On incube les tranches ainsi prélevées, pendant 75 minutes, à 24°C, dans du tampon Krebs saturé en O₂/CO₂ (95%/5%) et contenant du Fura-2/AM à la concentration de 7 µM. Après incubation, on rince les coupes plusieurs fois avec ce même tampon et on les laisse dans ce tampon jusqu'à leur utilisation. Pour mesurer la [Ca²⁺]ᵢ, on place les coupes, à 30°C, dans la cuve d'un spectrofluorimètre que l'on perfuse avec du tampon Krebs par l'intermédiaire d'une pompe. On réalise la dépolarisation des tranches en perfusant, pendant 3 minutes, du tampon Krebs contenant du KCl à 50 mM. On introduit le composé à tester dans le liquide de perfusion, 7 minutes après cette première dépolarisation et on réalise une seconde dépolarisation 7 minutes après l'introduction du composé à tester. On suit la fluorescence à deux longueurs d'onde d'excitation, 340 nm (forme liée au calcium) et 380 nm (forme libre), la longueur d'onde d'émission étant 510 nm. La [Ca²⁺]ᵢ est calculée selon la méthode décrite dans J. Biol. Chem., **260**, (1985), 3440-3450. L'effet inhibiteur des composés à tester est calculé par rapport à l'augmentation de la [Ca²⁺]ᵢ induite par du KCl 50 mM prise comme 100 %.
Le pourcentage d'inhibition de l'entrée du Ca²⁺, induit par les composés de l'invention, est dose-dépendant et se situe entre 10 et 50 %.

### Occlusion de l'artère cérébrale moyenne chez la souris

L'activité neuroprotectrice des composés de l'invention a été montrée dans un modèle d'ischémie focale par ligature de l'artère cérébrale moyenne chez la souris, selon une méthode analogue à celle décrite dans Brain Research, **522**, (1990), 290-307.
Six jours après occlusion de l'artère cérébrale moyenne par électrocoagulation sous anesthésie à l'halothane, on anesthésie de nouveau les souris et on prélève le cortex cérébral ipsilatéral à l'occlusion. Après homogénéisation du tissu, on évalue l'étendue de l'infarctus cérébral par mesure de l'augmentation de la densité des sites benzodiazépiniques périphériques (ω₃) à l'aide du composé [³H]PK 11195 de New England Nuclear. On administre les traitements curativement aux temps 5 minutes, 3 heures, 6 heures, 18 heures et 24 heures après l'occlusion par voie intrapéritonéale.
Les composés de l'invention diminuent la densité des sites benzodiazépiniques périphériques d'environ 40% à la dose de 10 mg/kg.

Les résultats des essais montrent que l'activité anti-ischémique des composés de l'invention est liée à leur effet inhibiteur sur les canaux calciques du tissu nerveux. Ils peuvent donc être utilisés dans la prophylaxie ou le traitement de certains états neuropathologiques, tels que notamment l'ischémie cérébrale, l'épilepsie, le vieillissement cérébral et les maladies neurodégénératives.

A cet effet ils peuvent être présentés sous toutes formes pharmaceutiques adaptées à l'administration entérale ou parentérale, en association avec des excipients appropriés, par exemple sous forme de comprimés, dragées, gélules, capsules, suppositoires, solutions ou suspensions buvables ou injectables, dosés pour permettre une administration journalière de 5 à 500 mg de substance active.

## Revendications

1. Composés répondant à la formule (I) 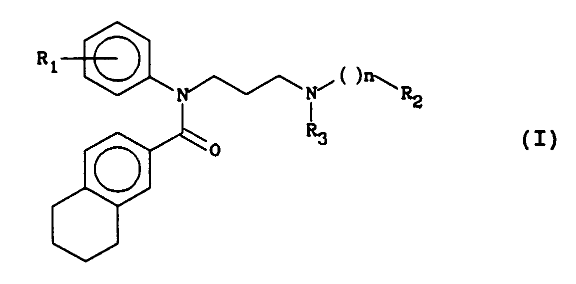 dans laquelle
R₁ représente soit un atome d'halogène, soit un groupe cyano, soit un groupe nitro, soit un groupe (C₁-C₆)alcoxy droit ou ramifié, soit un groupe (C₁-C₆)cycloalkyl(C₁-C₂)alcoxy, soit un groupe aryl(C₁-C₂)alcoxy,
R₂ représente soit un groupe (C₁-C₄)alkyle, soit un groupe aryle éventuellement substitué par un ou plusieurs groupes méthoxy, soit un groupe pyridinyle, soit un groupe benzodioxanyle,
R₃ représente soit un atome d'hydrogène, soit un groupe (C₁-C₂)alkyle, et
n = 0 à 3,
ainsi que leurs sels d'addition aux acides pharmaceutiquement acceptables.

2. Procédé de préparation des composés selon la revendication 1, caractérisé en ce que l'on fait réagir le 5,6,7,8-tétrahydronaphtaléne-2-carboxylate de méthyle avec une amine de formule (II) 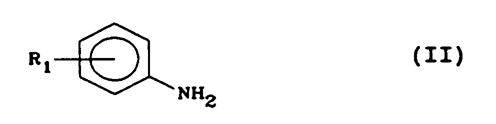 dans laquelle R₁ est tel que défini dans la revendication 1, pour obtenir un carboxamide de formule (IV) 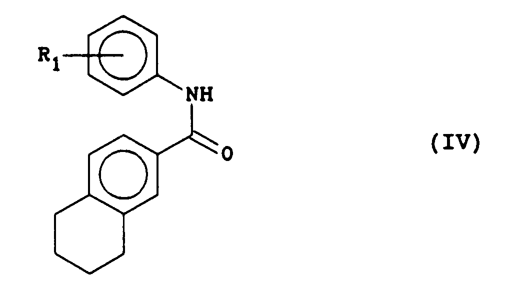 que l'on condense
* soit avec le 3-chloro-1,1-diéthoxypropane de formule (VIII) 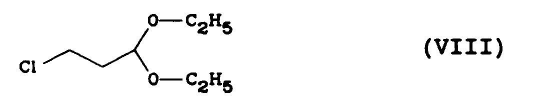 pour obtenir un carboxamide que l'on traite en milieu acide pour donner un carboxamide de formule (IX) 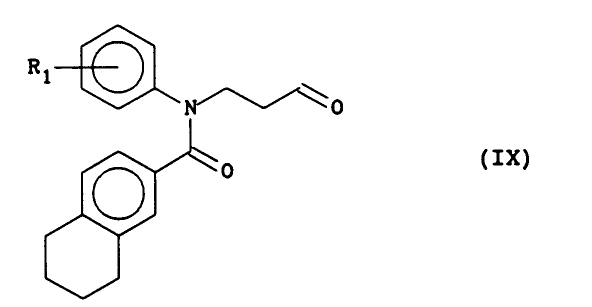 que l'on condense avec une amine de formule (X) 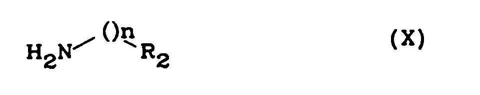 dans laquelle R₂ et n sont tels que définis dans la revendication 1, pour obtenir un carboxamide de formule (I bis) 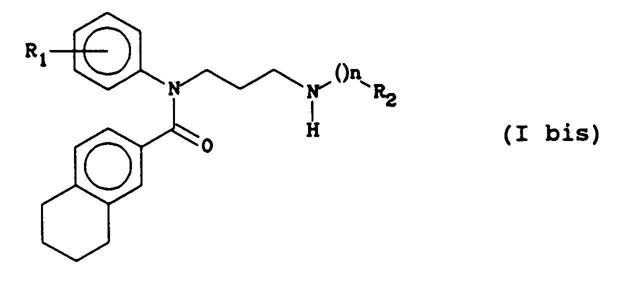
* soit avec du 1-bromo-3-chloropropane, en présence d'une base, pour obtenir un composé de formule (VI) 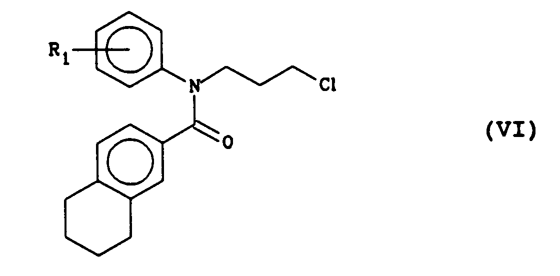 que l'on fait réagir avec une amine de formule (VII) 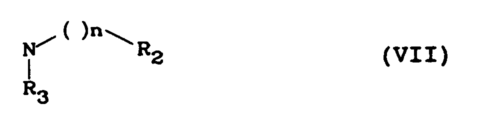
dans laquelle R₂, R₃ et n sont tels que définis dans la revendication 1,
pour obtenir le composé de formule générale (I)

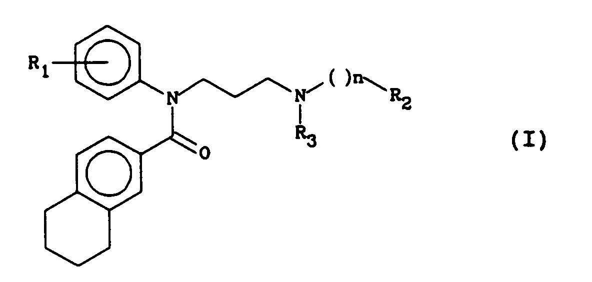

3. Médicament caractérisé en ce qu'il contient un composé selon la revendication 1.

4. Composition pharmaceutique caractérisée en ce qu'elle contient un composé selon la revendication 1 en association avec tout excipient approprié.

5. Composés répondant à la formule (XI) 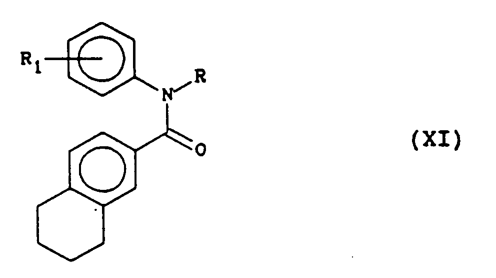 dans laquelle
R représente soit un atome d'hydrogène, soit un groupe 3-chloropropyle, soit un groupe 3-oxopropyle,
R₁ représente soit un atome d'halogène, soit un groupe cyano, soit un groupe nitro, soit un groupe (C₁-C₆)alcoxy droit ou ramifié, soit un groupe (C₁-C₆)cycloalkyl(C₁-C₂)alcoxy, soit un groupe aryl(C₁-C₂)alcoxy,
utiles pour la synthèse des composés selon la revendication 1.

## Claims

1. Compounds corresponding to the general formula (I) 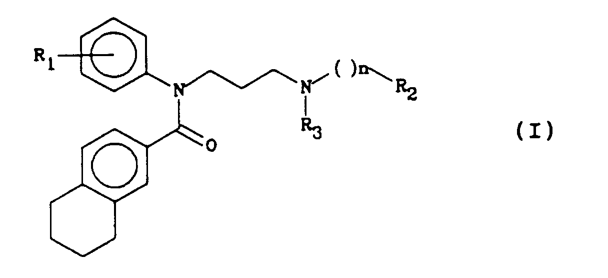 in which
R₁ represents either a halogen atom, or a cyano group, or a nitro group, or an unbranched or branched (C₁-C₆) alkoxy group, or a (C₁-C₆ cycloalkyl)(C₁-C₂ alkoxy) group, or an aryl(C₁-C₂ alkoxy) group,
R₂ represents either a (C₁-C₄) alkyl group, or an aryl group optionally substituted with one or more methoxy groups, or a pyridyl group, or a benzodioxanyl group,
R₃ represents either a hydrogen atom, or a (C₁-C₂) alkyl group, and
n = 0 to 3,
as well as their addition salts with pharmaceutically acceptable acids.

2. Process for preparing the compounds according to Claim 1, characterised in that methyl 5,6,7,8-tetrahydronaphthalene-2-carboxylate is reacted with an amine of formula (II) 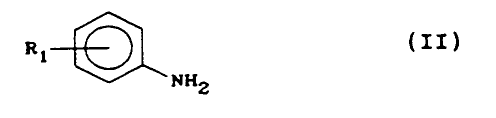 in which R₁ is as defined in Claim 1, to obtain a carboxamide of formula (IV) 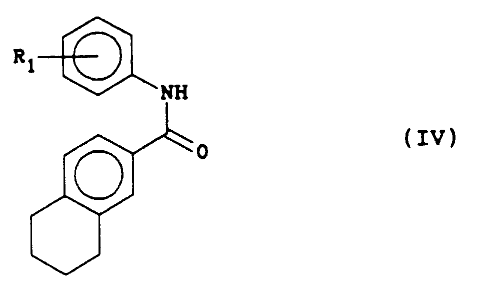 which is condensed
* either with 3-chloro-1,1-diethoxypropane of formula (VIII) 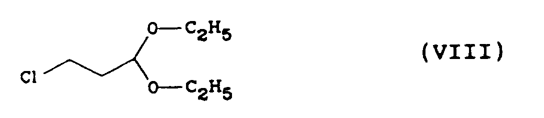 to obtain a carboxamide, which is treated in an acid medium to give a carboxamide of formula (IX) 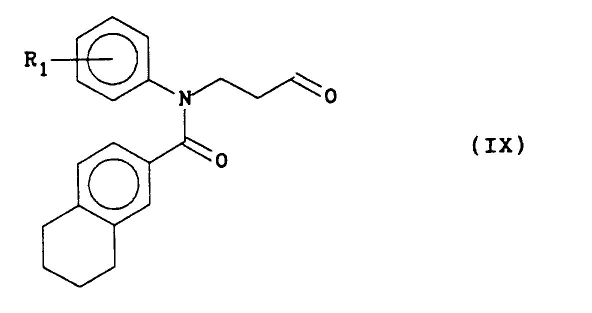 which is condensed with an amine of formula (X) 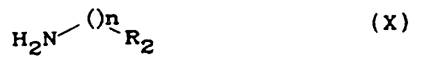 in which R₂ and n are as defined in Claim 1, to obtain a carboxamide of formula (I a) 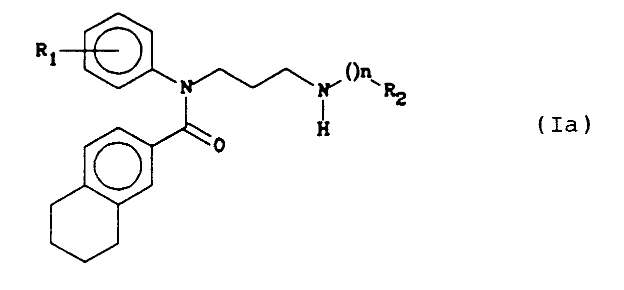
* or with 1-bromo-3-chloropropane, in the presence of a base, to obtain a compound of formula (VI) 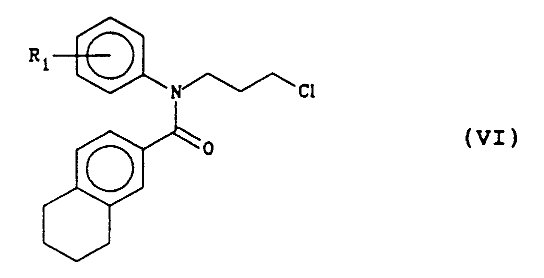 which is reacted with an amine of formula (VII) 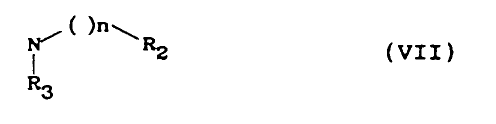 in which R₂, R₃ and n are as defined in Claim 1, to obtain the compound of general formula (I)

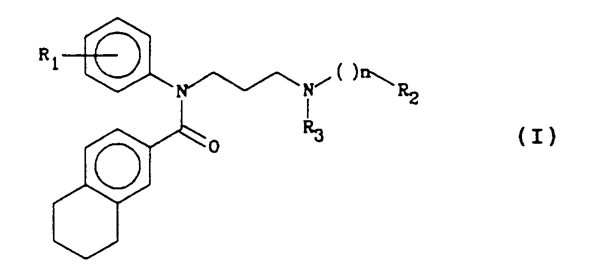

3. Medicinal product, characterised in that it contains a compound according to Claim 1.

4. Pharmaceutical composition, characterised in that it contains a compound according to Claim 1, in combination with any suitable excipient.

5. Compounds corresponding to the formula (XI) 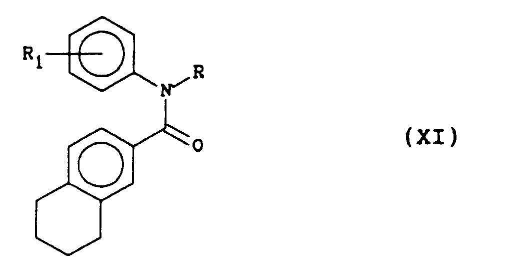 in which
R represents either a hydrogen atom, or a 3-chloropropyl group, or a 3-oxopropyl group, and
R₁ represents either a halogen atom, or a cyano group, or a nitro group, or an unbranched or branched (C₁-C₆) alkoxy group, or a (C₁-C₆ cycloalkyl)(C₁-C₂ alkoxy) group, or an aryl(C₁-C₂ alkoxy) group,
which are useful for the synthesis of the compounds according to Claim 1.

## Patentansprüche

1. Verbindungen der Formel (I) 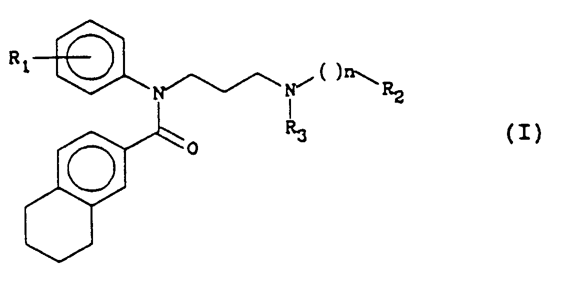 in der
R₁ entweder ein Halogenatom oder eine Cyanogruppe oder eine Nitrogruppe oder eine geradkettige oder verzweigte (C₁-C₆)-Alkoxygruppe oder eine (C₁-C₆)-Cycloalkyl-(C₁-C₂)-alkoxygruppe oder eine Aryl-(C₁-C₂)-alkoxygruppe,
R₂ entweder eine (C₁-C₄)-Alkylgruppe oder eine gegebenenfalls durch eine oder mehrere Methoxygruppen substituierte Arylgruppe oder eine Pyridinylgruppe oder eine Benzodioxanylgruppe,
R₃ entweder ein Wasserstoffatom oder eine (C₁-C₂)-Alkylgruppe und
n = 0 bis 3 bedeuten,
sowie deren Additionssalze mit pharmazeutisch annehmbaren Säuren.

2. Verfahren zur Herstellung der Verbindungen nach Anspruch 1, **dadurch gekennzeichnet**, daß man 5,6,7,8-Tetrahydronaphthalin-2-carbonsäuremethylester mit einem Amin der Formel (II) 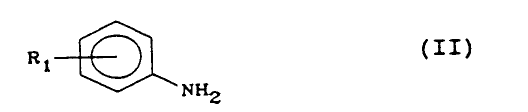 in der R₁ die in Anspruch 1 angegebenen Bedeutungen besitzt, umsetzt zur Bildung eines Carboxamids der Formel (IV) 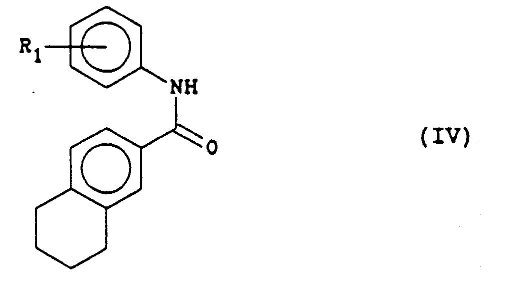 welche man
* entweder mit 3-Chlor-1,1-diethoxypropan der Formel (VIII) 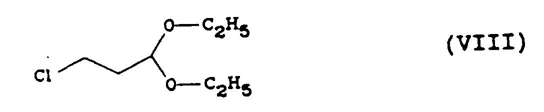 kondensiert zur Bildung eines Carboxamids, welches man in saurem Medium behandelt zur Bildung eines Carboxamids der Formel (IX) 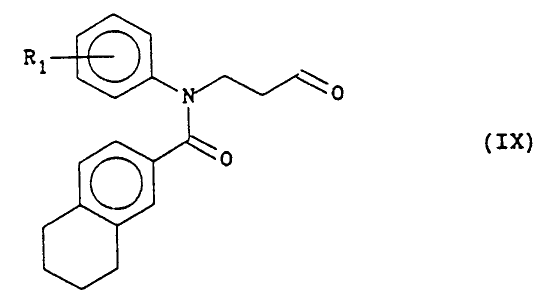 welches man mit einem Amin der Formel (X) 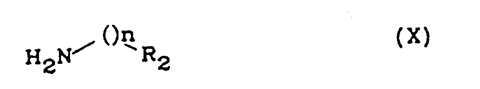 kondensiert, in der R₂ und n die in Anspruch 1 angegebenen Bedeutungen besitzen, zur Bildung eines Carboxamids der Formel (I bis) 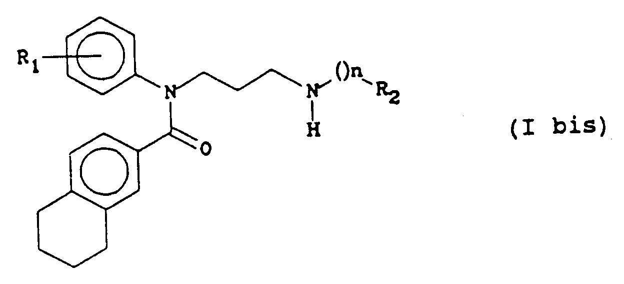
* oder mit 1-Brom-3-chlorpropan in Gegenwart einer Base kondensiert zur Bildung einer Verbindung der Formel (VI) 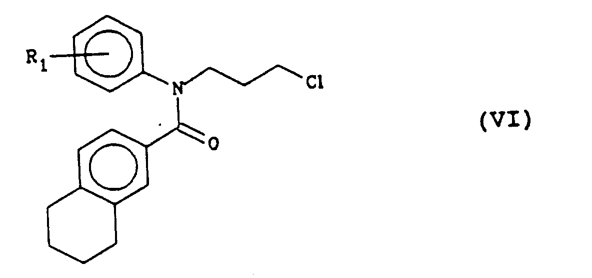 welche man mit einem Amin der Formel (VII) 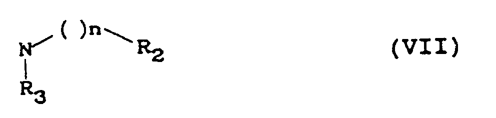 in der R₂, R₃und n die in Anspruch 1 angegebenen Bedeutungen besitzen, umsetzt zur Bildung der Verbindung der allgemeinen Formel (I)

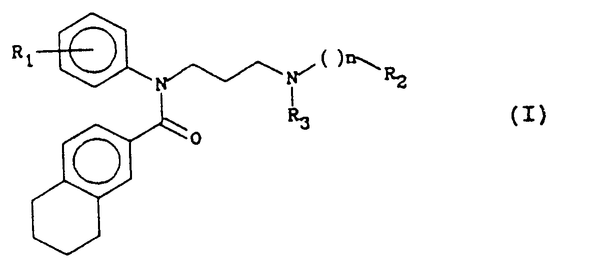

3. Arzneimittel, **dadurch gekennzeichnet**, daß es eine Verbindung nach Anspruch 1 enthält.

4. Pharmazeutische Zubereitung, **dadurch gekennzeichnet**, daß sie eine Verbindung nach Anspruch 1 in Kombination mit irgendeinem geeigneten Trägermaterial enthält.

5. Verbindungen der Formel (XI) 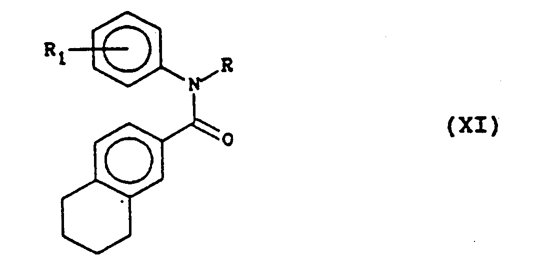 in der
R entweder ein Wasserstoffatom oder eine 3-Chlorpropylgruppe oder eine 3-Oxypropylgruppe,
R₁ entweder ein Halogenatom oder eine Cyanogruppe oder eine Nitrogruppe oder eine geradkettige oder verzweigte (C₁-C₆)-Alkoxygruppe oder eine (C₁-C₆)-Cycloalkyl-(C₁-C₂)-alkoxygruppe oder eine Aryl-(C₁-C₂)-alkoxygruppe bedeuten,
als Zwischenprodukte für die Synthese der Verbindungen nach Anspruch 1.
